# EUROPEAN PATENT APPLICATION

(11) **EP 4 613 777 A1**
(43) Date of publication of application: **10.09.2025**
(21) Application number: 23910075.3
(22) Date of filing: 11.12.2023
(51) Int. Cl.: C07K 16/40, C12N 15/13, C12N 9/12, G01N 33/573

(54) **IMMUNE-CELL-TARGETING ANTIBODY OR FUNCTIONAL FRAGMENT THEREOF, AND APPLICATION THEREOF**

(30) Priority: 29.12.2022 CN 202211710279
(71) Applicant: Guangdong Keyangle Life Technology Co., Ltd., Jiangmen, Guangdong 529000 (CN)
(72) Inventor: WEI, Yuan'an, Jiangmen, Guangdong 529000 (CN); LI, Yueming, Jiangmen, Guangdong 529000 (CN)
(74) Representative: Clark, Claudia
(86) International application number: PCT/CN2023/137916
(87) International publication number: WO 2024/140145

(57) **Abstract**

An immune-cell-targeting antibody or a functional fragment thereof, and an application thereof. The antibody or the functional fragment thereof targets and identifies a Triokinase/FMN cyclase C-terminus fragment sequence. The antibody can be directly used for tagging and identifying immune cells. After the antibody and an antigen polypeptide thereof combine to form a self-assembled complex, the immune cell identification effect thereof is significantly improved, and in related applications can exhibit a decreased lower detection limit, thereby greatly improving identification capabilities and detection sensitivity for various immune cells. Moreover, a probe obtained on the basis of the antibody can likewise carry various modification groups and reporter groups, and can thus be used in approaches to different application scenarios, such as flow cytometry, immunofluorescence imaging, immunoassays, detection chips, etc., and has flexible and broad applicability and scope of application.

## Description

### TECHNICAL FIELD

The present disclosure belongs to the technical field of molecular immunology, and in particular relates to an antibody or its functional fragment targeting immune cells, and applications thereof.

### BACKGROUND

Immune cells refer to cells that participate in or are associated with immune responses, including lymphocytes, dendritic cells, monocytes/macrophages, granulocytes, mast cells, etc. Immune cells can be divided into many types. In the human body, various immune cells serving distinct roles and perform different functions; and they influence and interact with each other to form a complex human immune regulatory network. For example, mature monocytes/macrophages have strong mobility in the body. After phagocytosing bacteria, pathogens and dead cells in relevant tissues, monocytes/macrophages can enter lymph nodes or lymphatic organs such as the spleen through nearby lymphatic vessels and present the antigens of the engulfed substances to other lymphocytes, such as T cells or B cells. Therefore, rapid and sensitive detection of immune cell types and their functions is of great significance to the determination of human immune balance and immune-related conditions.

Currently, clinical testing of immune cells includes immune cell counting, detection and classification of immune cell subtypes, and detection of cytokines. Detection and classification of immune cell subtypes is to perform immunophenotyping on cells. For example, peripheral blood lymphocyte subsets are roughly divided into three groups based on biological functions and cell surface antigen expression: T lymphocytes (CD3+), B lymphocytes (CD19+) and NK lymphocytes (CD3-CD16+ and/or CD56+). Immunophenotyping refers to the analysis of heterogeneous cell populations to identify the presence and proportion of multiple target cell populations. This process is mostly performed using monoclonal antibodies, which can specifically detect specific antigens expressed by these cells, i.e., biomarkers. These markers are often functional membrane proteins involved in cell communication, adhesion or metabolism. Flow cytometry remains the gold standard for immunophenotyping. It can use multiple antibodies (each labeled with a different fluorescent dye) simultaneously to quickly detect and evaluate the specific expression of cell markers, allowing specific immune cell subsets to be identified and quantified. This technology has been widely used in basic research and clinical laboratories. Currently, most immune cell markers belong to the Cluster of Differentiation (CD) classification system, with CD-specific antibodies are widely used to detect specific immune cell populations and subpopulations. The present invention provides novel antibodies that effectively identify specific phenotypes of immune cells and antibody-based self-assembly probes.

Prior granted Chinese patents CN111051504A and CN110760491A of the applicant respectively disclose a polypeptide suitable for identifying monocytes/macrophages, which can automatically target and recognize macrophages in different anatomical parts in living animals and aggregate and adhere to macrophages. If the polypeptide carries imaging or reporting groups, various monocytes/macrophages can be imaged *in situ,* clearly showing the *in vivo* distribution of related monocytes/macrophages and their temporal and spatial change relationships, thereby studying the physiological functions of monocytes/macrophages *in vivo* and their real-time distribution. Based on these polypeptides, the applicant has constructed a class of antibodies that can efficiently recognize immune cells, and further developed antibody-peptide self-assembly probes with higher sensitivity based on such type of antibodies, which can achieve recognition, differentiation, targeted tracing and localization of different states of immune cells by means of simple selection and assembly of antigen polypeptides and different labeled antibodies.

### SUMMARY

In order to achieve the above objectives, an immune-cell-targeting antibody or a functional fragment thereof and use thereof are proposed. The antibody is obtained by immunization with the C-terminal fragment sequence of Triokinase/FMN cyclase containing VLQ motif as an antigen. The antibody can specifically target immune cells in addition to specifically binding to an antigen thereof. Moreover, the recognition effect is not interfered by the carried reporting groups or other substances, and stable, efficient and accurate targeting can be achieved. Furthermore, after the antibody or the functional fragment thereof in the present disclosure self-assembles the antigen, the recognition effect is much stronger than that of a single antibody or antigen on immune cells, showing extremely broad application prospects.

In a first aspect of the present disclosure, an antibody or a functional fragment thereof is provided, which targets and recognizes a C-terminal fragment sequence of Triokinase/FMN cyclase.

In some embodiments of the present disclosure, the antibody is produced by immunizing with the C-terminal fragment sequence of Triokinase/FMN cyclase as the immunogen.

In some embodiments of the present disclosure, the C-terminal fragment sequence of Triokinase/FMN cyclase includes:
(a) an amino acid sequence containing VLQ motif;
(b) an amino acid sequence exhibiting >80% sequence identity to (a), wherein said sequence is derived from non-human species and maintains immunogenic properties; and
(c) a variant of the amino acid sequence in (a) or (b).

In some embodiments of the present disclosure, the amino acid sequence in (b) is 80%, 85%, 90% or more identical to the amino acid sequence in (a).

In some embodiments of the present disclosure, the amino acid sequence in (b) is 80%, 81%, 82%, 83%, 84%, 85%, 86%, 87%, 88%, 89%, 90%, 91%, 92%, 93%, 94%, 95%, 96%, 97%, 98%, 99% or more identical to the amino acid sequence in (a).

In some embodiments of the present disclosure, the amino acid sequence in (b) can be obtained by modifying or substituting one or more amino acids in the amino acid sequence in (a). The specific method of modification and substitution can use any method known in the prior art.

In some embodiments of the present disclosure, the amino acid sequences in (a), (b) and (c) include 45 or less amino acid residues. In some embodiments of the present disclosure, the amino acid sequences in (a), (b) and (c) include 41 or less amino acid residues.

In some embodiments of the present disclosure, the amino acid sequences in (a), (b) and (c) include 9 to 41 amino acid residues.

In some embodiments of the present disclosure, the C-terminal fragment sequence of Triokinase/FMN cyclase described in (a) includes an amino acid sequence having more than 80% identity with the amino acid sequence described in SEQ ID NOs: 2 to 18, and 29.

In some embodiments of the present disclosure, the amino acid sequence having more than 80% identity with the amino acid sequence described in SEQ ID NOs: 2 to 18, and 29 includes a human sequence and a non-human animal sequence, and both contain VLQ motif.

In some embodiments of the present disclosure, the amino acid sequence is a human sequence and a non-human animal sequence having 80%, 85%, 90% or more identity with the amino acid sequence described in SEQ ID NOs: 2 to 18, and 29, and both contain VLQ motif.

In some embodiments of the present disclosure, the amino acid sequence is a human sequence and a non-human animal sequence having 80%, 81%, 82%, 83%, 84%, 85%, 86%, 87%, 88%, 89%, 90%, 91%, 92%, 93%, 94%, 95%, 96%, 97%, 98%, 99% or more identity with the amino acid sequence described in SEQ ID NOs: 2 to 18, 29, and both contain VLQ motif.

In a particular embodiment of the present disclosure, for the species source of the Triokinase/FMN cyclase and the "animal" mentioned above, there is no particular limitation on the species of animals, for example, including birds and mammals. Examples of birds include poultry such as chickens and ducks; examples of mammals include mice, rats, rabbits, pigs, dogs, cattle, primates (e.g., humans), and the like. Non-human animals specifically refer to mammals and birds other than humans. In other words, the polypeptide sequence at the C-terminus of the Triokinase/FMN cyclase is a relatively conservative sequence at least in mammals and birds, and mutations of amino acid residues at certain sites do not affect its function of targeting immune cells.

In some embodiments of the present disclosure, the amino acid sequence having more than 80% identity with the amino acid sequence described in SEQ ID NOs: 2 to 18, and 29 includes LDQPDPGAVAAAAIFRAILEVLQTQGA (SEQ ID NO: 7) and EQPDPGAVAAAAILRAILEVLQS (SEQ ID NO: 12). Based on the SEQ ID NO: 7 and the SEQ ID NO: 12, the antibody KA-001 and the antibody KA-004 are obtained, respectively.

In some embodiments of the present disclosure, the amino acid sequence having more than 80% identity with the amino acid sequence described in SEQ ID NOs: 2 to 18, and 29 can be obtained by modifying, substituting, inserting or deleting one or more amino acids in the amino acid sequence described in SEQ ID NOs: 2 to 18, and 29. The specific method of the modification, substitution, insertion or deletion can use any method known in the prior art.

In some embodiments of the present disclosure, the C-terminal fragment sequence of Triokinase/FMN cyclase described in (b) includes SEQ ID NO: 1 or an amino acid sequence having more than 80% identity with the amino acid sequence described in SEQ ID NO: 1.

In some embodiments of the present disclosure, the amino acid sequence having more than 80% identity with the amino acid sequence described in SEQ ID NOs: 2 to 18, and 29 includes less than 45 amino acid residues. In some embodiments of the present disclosure, the amino acid sequence in (d) may include 45, 44, 43, 42 or 41 or less amino acid residues.

In some embodiments of the present disclosure, the amino acid sequence having more than 80% identity with the amino acid sequence described in SEQ ID NOs: 2 to 18, and 29 comprises 41 or less amino acid residues. The amino acid sequence having more than 80% identity with the amino acid sequence described in SEQ ID NOs: 2 to 18, 29 includes 9 to 41 amino acid residues. It may include 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41 amino acid residues.

In the present disclosure, the term "Triokinase/FMN cyclase" refers to an enzyme that catalyzes the phosphorylation of dihydroxyacetone and glyceraldehyde and the cleavage of ribonucleoside diphosphate-X compounds, of which FAD is the optimal substrate. Triokinase/FMN cyclase (also known as DAK protein) was first discovered in prokaryotic/eukaryotic microorganisms (refer to Erni B, Siebold C, Christen S, et al. Small substrate, big surprise: Fold, function and phylogeny of dihydroxyacetone kinases[J]. Cellular and Molecular Life Sciences, 2006, 63(7-8): 890-900), and was reported to catalyze the phosphorylation of dihydroxyacetone to produce dihydroxyacetone phosphate (Dha-P). In 2005, it was discovered that FAD-AMP lyase (also known as FMN cyclase) found in rat liver extracts had homology with the amino acid sequence of microbial DAK protein, and it was confirmed that DAK protein has the dual functional characteristics of kinase and cyclase. In 2007, it was reported that DAK protein can inhibit RNA helicase MDA5-mediated cellular antiviral signal transduction (refer to Diao F, Li S, Tian Y, et al. Negative regulation of MDA5-but not rig-i-mediated innate antiviral signaling by the dihydroxyacetone kinase[J]. Proceedings of the National Academy of Sciences, 2007, 104(28): 11706-11711.).

In the Uniprot protein database, the latest name of DAK protein is Triokinase/FMN cyclase. It has been found that the C-terminal fragment of Triokinase/FMN cyclase is highly conserved in animals, especially in mammals (including humans) (A. Cabezas et al., Identification of human and rat FAD-AMP lyase (cyclic FMN forming) as ATP-dependent dihydroxyacetone kinases, Biochemical and Biophysical Research Communications, 2005, 338: 1682-1689). The C-termini of Triokinase/FMN cyclase proteins from 20 mammals (including humans, gorillas, monkeys and other primates) were found to have 100% sequence identity in the Uniprot database. The gene encoding human Triokinase/FMN cyclase is located on chromosome 11, region 11q 12.2, with an access number of DQ138290 in GenBank and a gene identification number of gene ID: 26007 (also numbered DKFZP586B1621).

In addition, according to a particular embodiment of the present disclosure, the selection of the polypeptide of the C-terminal fragment of Triokinase/FMN cyclase (i.e., the polypeptide targeting and recognizing immune cells) in the present disclosure is mainly based on the inventor's prior Chinese patents CN 110885805 A, CN 111051504 A and CN 110760491 A, including but not limited to the sequences shown in Table 1 of the specification.

It should be noted that the present disclosure does not claim to protect the polypeptide of the C-terminal fragment of Triokinase/FMN cyclase (i.e., the polypeptide that targets and recognizes immune cells), which is only introduced into the present disclosure as one of the necessary materials for preparing the antibodies and probes in the present disclosure.

In some embodiments of the present disclosure, the antibody is an antibody prepared by using the polypeptide of the C-terminal fragment of Triokinase/FMN cyclase (i.e., the polypeptide that targets and recognizes immune cells) as an immune antigen. The functional fragment refers to a truncated fragment that can realize the functionality of the antibody. Furthermore, the functional fragment is an antibody or truncated fragment that can realize the function of the antibody prepared by genetic engineering or antibody engineering.

In some embodiments of the present disclosure, the antibody is a monoclonal antibody that is prepared by a conventional hybridoma approach and screened.

In some embodiments of the present disclosure, the antibody is an antibody prepared by antibody engineering based on the CDR sequence determined above.

In some embodiments of the present disclosure, the variant described in (c) includes a complex polypeptide obtained by ligating the C-terminal fragment sequence of Triokinase/FMN cyclase with an adapter sequence, a marker, a tag, an amino acid sequence or a carrier portion, and the variants are all immunogenic.

In some embodiments of the present disclosure, the carrier portion includes a protein carrier, and the protein carrier includes hemocyanin (KLH), serum albumin (ALB), and ovalbumin (OVA).

In some embodiments of the present disclosure, the serum albumin includes bovine serum albumin (BSA), human serum albumin (HSA), rabbit serum albumin (RSA), equine serum albumin (ESA), and goat serum albumin (GSA).

In some embodiments of the present disclosure, the protein carrier is coupled to the side chain thiol of the adapter amino acid Cys which is artificially designed and added to the C-terminal fragment sequence of Triokinase/FMN cyclase.

In some embodiments of the present disclosure, variants obtained by processing antigens, such as hapten conjugates and artificial antigens. The antigens, which also contain complete effective epitope VLQ, have the ability to target immune cells when used as immunogens to obtain antibodies through routine immunization.

In some embodiments of the present disclosure, the artificial antigen includes the artificial antigen obtained by coupling the aforementioned targeting polypeptide with a protein carrier via an adapter amino acid (such as cysteine-Cys).

In a particular embodiment of the present disclosure, the antibody can use VLQ motif as a recognition epitope to form a self-assembled composition with polypeptides of various chain lengths that target and recognize immune cells and contain the recognition epitope, and use the self-assembled composition as a probe and an immune cell marker to image and perform flow cytometric analysis on the immune cells.

In a particular embodiment of the present disclosure, the results of ELISA antigen epitope detection show that the antibodies KA-001 and KA-004 of the present disclosure can specifically recognize the epitope composed of the VLQ sequence at the C-terminus of Triokinase/FMN cyclase.

In a particular embodiment of the present disclosure, the results of ELISA antigen epitope detection show that the antibodies KA-001 and KA-004 of the present disclosure can specifically recognize the epitope composed of the VLQS or VL sequence at the C-terminus of Triokinase/FMN cyclase.

In a particular embodiment of the present disclosure, after self-assembly with its immune antigen, the antibody of the present disclosure will have some functions different from those of a directly labeled antibody, for example, the antibody can recognize not only monocytes and neutrophils, but also B lymphocytes. Furthermore, its recognition effect is far better than the single use of antibodies or recognition peptides.

In some embodiments of the present disclosure, the amino acid sequences of the complementarity determining regions (CDRs) of the heavy chain variable regions of the antibody (KA-001) are shown in the amino acid sequences at positions 26 to 35, 50 to 66, and 99 to 110 of SEQ ID NO: 19, respectively; the amino acid sequences of the complementarity determining regions (CDRs) of the light chain variable regions are shown in amino acid sequences at positions 24 to 34, 50 to 56, and 89 to 97 of SEQ ID NO: 21, respectively.

In some embodiments of the present disclosure, the heavy chain variable region of the antibody (KA-001) is shown in SEQ ID NO: 19; the light chain variable region is shown in SEQ ID NO: 21.

In some embodiments of the present disclosure, the antibody further contains a constant region.

In some embodiments of the present disclosure, the constant region is a human constant region.

In some embodiments of the present disclosure, the antibody is prepared by using at least one amino acid sequence having at least 95% identity with the amino acid sequence described in SEQ ID NOs: 1 to 18, and 29 as an immune antigen.

In some embodiments of the present disclosure, the amino acid sequence of the antigen has 95%, 96%, 97%, 98%, 99% or more identity with the amino acid sequence described in SEQ ID NOs: 1 to 18, and 29.

For the selection criteria of the amino acid sequence having at least 95% identity with the amino acid sequence described in SEQ ID NOs: 1 to 18, reference can be made to the inventor's prior Chinese patents CN 110885805 A, CN 111051504 A and CN 110760491 A, which include but are not limited to the sequences shown in Table 1 of the present specification.

In some embodiments of the present disclosure, the antibody is modified or linked with a small-molecule active substance.

In some embodiments of the present disclosure, the small-molecule active substance includes a polypeptide adapter, a biotin-avidin system, a colorimetric enzyme, a fluorescent labeling group, a chemiluminescent labeling group, an isotope or a magnetic functional group.

In some embodiments of the present disclosure, the adapter includes lysine (K) or cysteine (C).

The colorimetric enzyme includes peroxidase and alkaline phosphatase.

The fluorescent labeling group includes fluorescent proteins, rhodamines, fluoresceins, iodocyanins, cyanine dyes, Alexa Fluor dyes and/or quantum dot fluorescent groups.

The chemiluminescent labeling group includes an acridinium ester compound.

The magnetic functional group includes a group capable of magnetic resonance imaging and changing relaxation efficiency.

The isotope includes a radionuclide.

In some embodiments of the present disclosure, the radionuclide includes a radionuclide used in radioimmunoimaging and radioimmunotherapy.

In some embodiments of the present disclosure, the antibody targets and recognizes immune cells.

In some embodiments of the present disclosure, the immune cells include but are not limited to lymphocytes, dendritic cells, monocyte precursors, monocytes/macrophages, neutrophils, basophils, eosinophils, and mast cells.

In a second aspect of the present disclosure, a nucleic acid molecule encoding the antibody or the functional fragment thereof described in the first aspect of the present disclosure is provided.

In some embodiments of the present disclosure, the nucleic acid molecule includes the nucleotide sequence of any one of the antibodies or the functional fragments thereof described in the first aspect of the present disclosure or a combination of the nucleotide sequences of multiple antibodies or functional fragments thereof.

In some embodiments of the present disclosure, the nucleic acid molecule includes a nucleic acid molecule encoding the heavy chain and/or light chain of the antibody or the functional fragment thereof described in the first aspect of the present disclosure.

In some embodiments of the present disclosure, the nucleic acid molecule encoding the heavy chain of the antibody or the functional fragment thereof described in the first aspect of the present disclosure includes but is not limited to a sequence containing the sequence (KA-001) shown in SEQ ID NO: 20 or a sequence of a functional fragment thereof.

In some embodiments of the present disclosure, the nucleic acid molecule encoding the light chain of the antibody or the functional fragment thereof described in the first aspect of the present disclosure includes but is not limited to a sequence containing the sequence (KA-001) shown in SEQ ID NO: 22 or a sequence of a functional fragment thereof.

In some embodiments of the present disclosure, the functional fragment is a nucleotide fragment that determines its ability to bind to an antigen, including a nucleic acid molecule encoding an antibody CDR region or a variant thereof.

In some embodiments of the present disclosure, the functional fragment is a nucleic acid molecule or a variant sequence thereof that encodes at least one CDR region on the light chain and/or heavy chain of the antibody described in the first aspect of the present disclosure.

In a third aspect, the present disclosure provides an expression vector or a transformer containing the nucleic acid molecule described in the second aspect of the present disclosure.

In some embodiments of the present disclosure, the transformant includes any host cell that can express the nucleic acid molecule.

In some embodiments of the present disclosure, the transformant includes any transformant containing the expression vector described in the third aspect of the present disclosure.

In some embodiments of the present disclosure, those skilled in the art can, based on the present disclosure, culture the aforementioned host cells of the present disclosure under conditions that allow the production of the antibodies or the functional fragments thereof described in the first aspect of the present disclosure, and recover the antibodies or the functional fragments thereof produced in such way.

In a fourth aspect, the present disclosure provides a probe targeting immune cells, the probe includes the antibody or the functional fragment thereof and the antigen polypeptide thereof described in the first aspect of the present disclosure.

In a particular embodiment of the present disclosure, the antibody described in the first aspect of the present disclosure can be used alone as a probe targeting immune cells, and can be used as a probe targeting immune cells based on further self-assembly in combination with a polypeptide that targets and recognizes immune cells to form a self-assembled composition.

In some embodiments of the present disclosure, the antibody and the antigen polypeptide thereof can enhance the antibody's ability to recognize and sensitivity to immune cells after self-assembly.

In some embodiments of the present disclosure, the antibody and the antigen polypeptide thereof are mixed at room temperature to form an immune cell self-assembled probe.

In some embodiments of the present disclosure, a small-molecule active substance is linked to the probe.

In some embodiments of the present disclosure, the small-molecule active substance includes a polypeptide adapter, a biotin-avidin system, a colorimetric enzyme, a fluorescent labeling group, a chemiluminescent labeling group, an isotope or a magnetic functional group.

In some embodiments of the present disclosure, the adapter includes lysine (K) or cysteine (C).

In some embodiments of the present disclosure, the colorimetric enzyme includes peroxidase and alkaline phosphatase; the fluorescent labeling group includes fluorescent proteins, rhodamines, fluoresceins, iodocyanins, cyanine dyes, Alexa Fluor dyes and/or quantum dot fluorescent groups; the magnetic functional group includes a group capable of magnetic resonance imaging and changing relaxation efficiency; the isotope includes a radionuclide.

In some embodiments of the present disclosure, the radionuclide includes a radionuclide used in radioimmunoimaging and radioimmunotherapy.

In some embodiments of the present disclosure, the probe targets and recognizes immune cells.

In some embodiments of the present disclosure, the immune cells include lymphocytes, dendritic cells, monocyte precursors, monocytes/macrophages, neutrophils, basophils, eosinophils, and mast cells.

In a particular embodiment of the present disclosure, the inventors have discovered that the probe obtained by binding an antibody to a polypeptide specifically recognized by the antibody can identify and label monocytes, neutrophils and some lymphocyte subsets in white blood cells (WBC) from different animal sources in flow cytometric analysis.

In a particular embodiment of the present disclosure, for the species source of the Triokinase/FMN cyclase and the "animal" mentioned above, there is no particular limitation on the species of animals, for example, including birds and mammals. Examples of birds include poultry such as chickens and ducks; examples of mammals include mice, rats, rabbits, pigs, dogs, cattle, primates (e.g., humans), and the like.

In a particular embodiment of the present disclosure, the probe has similar recognition and staining capabilities for WBC immune cells of humans and mice, but the fluorescence response intensity of the same cell population is different between humans and mice.

In a fifth aspect, the present disclosure provides use of the antibody or the functional fragment thereof described in the first aspect of the present disclosure, the nucleic acid molecule described in the second aspect, or the expression vector or transformant described in the third aspect in the preparation of a product targeting immune cells.

In some embodiments of the present disclosure, the product includes a probe, a detection reagent, a detection kit, and a detection chip.

In some embodiments of the present disclosure, the product further includes other auxiliary agents, including but not limited to solvents, culture media, biological dyes, and coupling agents.

In some embodiments of the present disclosure, the immune cells include lymphocytes, dendritic cells, monocyte precursors, monocytes/macrophages, neutrophils, basophils, eosinophils, and mast cells.

In a particular embodiment of the present disclosure, the product can be used for targeted recognition of immune cells.

In a particular embodiment of the present disclosure, an effective amount of the above product is administered to a subject in need thereof so as to specifically bind to immune cells in the subject.

In a sixth aspect, the present disclosure provides a method for preparing the antibody or the functional fragment thereof described in the first aspect of the present disclosure, including the following steps:
designing, synthesizing and preparing a synthetic antigen based on the C-terminal fragment sequence of Triokinase/FMN cyclase, and obtaining an antibody by immunizing an animal with the synthetic antigen; or
preparing an antibody or a functional fragment by using the nucleic acid molecule described in the second aspect or the expression vector or transformant described in the third aspect through genetic engineering methods or antibody engineering.

In some particular embodiments of the present disclosure, the C-terminal fragment sequence of the Triokinase/FMN cyclase is:
LDQPDPGAVAAAAIFRAILEVLQTQGA (SEQ ID NO: 7) or a variant thereof.

In some embodiments of the present disclosure, the variant includes an amino acid sequence having more than 80% identity with the amino acid sequence described in SEQ ID NO: 7.

In some embodiments of the present disclosure, the amino acid sequence includes 41 or less amino acid residues.

In some embodiments of the present disclosure, the variant includes a complex polypeptide obtained by ligating a C-terminal fragment sequence of Triokinase/FMN cyclase to an adapter sequence, a marker, a tag, an amino acid sequence or a carrier portion, and the variants are all immunogenic.

In some embodiments of the present disclosure, the carrier portion includes a protein carrier.

In some embodiments of the present disclosure, the nucleic acid molecule includes a nucleotide sequence of any one of the antibodies described in the first aspect of the present disclosure or a combination of nucleotide sequences of multiple antibodies.

In some embodiments of the present disclosure, the nucleic acid molecule includes a nucleic acid molecule encoding a heavy chain and/or a light chain of the antibody described in the first aspect of the present disclosure.

In the present disclosure, the antibody or the functional fragment thereof prepared by the method described in the sixth aspect of the present disclosure also fall within the scope of protection of the present disclosure.

The present disclosure achieves the following beneficial effects:
1. The antibody of the present disclosure is prepared based on a polypeptide capable of recognizing immune cells as an antigen. It can not only be used in combination with an antigen polypeptide, but can also be used as a direct-labeled antibody to directly mark and recognize immune cells, with a good recognition effect and an extremely high application value.
2. The antibody of the present disclosure has a significantly enhanced recognition effect after binding to an antigen polypeptide, and has a better effect for analysis of specific immune cells, and can exhibit stronger fluorescence intensity in related applications, thereby greatly improving the recognition ability and detection sensitivity of different immune cells.
3. The probe obtained on the basis of the antibody can carry various modification groups and reporting groups, and can thus be used in approaches to different application scenarios, and has flexible and broad applicability and scope of application.

### BRIEF DESCRIPTION OF DRAWINGS

FIG. 1 is a plasmid map of pcDNA3.4 in the example of the present disclosure, where A is a plasmid map of a heavy chain and B is a plasmid map of a light chain.
FIG. 2 is a comparison diagram of ELISA results of KA-001 and KA-004 specifically binding to different peptide fragments in the example of the present disclosure.
FIG. 3 is a flow cytometry comparison diagram of the targeting labeling effect of a fluorescent direct-labeled antibody (KA-001) alone and in combination with a target recognition polypeptide as an immune cell targeting probe.
FIG. 4 is a fluorescence intensity comparison diagram corresponding to FIG. 3.
FIG. 5 is a flow cytometry comparison diagram of the targeting labeling effect of a fluorescent direct-labeled antibody (KA-004) alone and in combination with a target recognition polypeptide as an immune cell targeting probe.
FIG. 6 is a fluorescence intensity comparison diagram corresponding to FIG. 5.

### DETAILED DESCRIPTION

The present disclosure is described in detail below in conjunction with specific examples. However, it should be understood that the present disclosure is not limited to the following specific examples. The protection scope of the present disclosure is defined by the appended claims, and the following examples can be amended and combined arbitrarily within the protection scope.

### Example 1 Polypeptides and antibodies targeting and recognizing immune cells

The polypeptides used in the example of the present disclosure were synthesized by a conventional solid-phase polypeptide chemical synthesis method using a CEM fully automatic microwave polypeptide synthesizer according to the instructions provided by the instrument supplier.

For the polypeptides targeting and recognizing immune cells in the examples of the present disclosure, reference can be made to the prior Chinese patents CN 110885805 A, CN 111051504 A and CN 110760491 A, including but not limited to the sequences shown in Table 1 below.

**Table 1 Polypeptides targeting and recognizing immune cells from different animal sources**

| Sequence No. | Sequence | Species |
|---|---|---|
| SEQ ID NO:1 | LQPDPGAVAAAAVLRAVLEGLQG-**C**-X | *Gallus gallus* |
| SEQ ID NO:2 | DQPDPGAVAAAAIFRAILEVLQTKAA-**C**-X | *Rattus norvegicus* |
| SEQ ID NO:3 | DQPDPGAVAAAAILRTILEVLQSQGV-**C**-X | *Canis lupus* |
| SEQ ID NO:4 | DQPDPGAVAAAAILRAILEVLQSQGA-**C**-X | *Sus scrofa* |
| SEQ ID NO:5 | DQPDPGAVAAAAILRAILEVLQSQGA-**C**-X | *Bos taurus* |
| SEQ ID NO:6 | EQPDPGAVAAAAILRAILEVLQS-**C**-X | *Macaca mulatta* |
| SEQ ID NO:7 | LDQPDPGAVAAAAIFRAILEVLQTQGA-**C**-X | Mouse |
| SEQ ID NO:8 | AILEVLQS-**C**-X | *Homo sapiens* |
| SEQ ID NO:9 | LRAILEVLQS-**C**-X | |
| SEQ ID NO:10 | ILRAILEVLQS-**C**-X | |
| SEQ ID NO:11 | AAILRAILEVLQS-**C**-X | |
| SEQ ID NO:12 | EQPDPGAVAAAAILRAILEVLQS-**C**-X | |
| SEQ ID NO: 13 | PGAVAAAAILRAILEVLQS-**C**-X | |
| SEQ ID NO:14 | KNMEAGAGRASYISSARLEQPDPGAVAAAAILRAILEVLQS -C-X | |
| SEQ ID NO:15 | AVLEVLQG-**C**-X | Modified sequence |
| SEQ ID NO:16 | VLRAVLEVLQG-**C**-X | |
| SEQ ID NO:17 | EQPDPSAVAAAAILRAILEVLQG-**C**-X | |
| SEQ ID NO:18 | LQPDPSAVAAAAVLRAVLEVLQG-**C**-X | |

The underlined and bold part (**C**) was an adapter, and X represented a marker, a tag, or a carrier portion, where the carrier included OVA, BSA, and KLH.

The modified polypeptide targeting and recognizing immune cells was used as an immune antigen to prepare an antibody. Antibodies include monoclonal antibodies and polyclonal antibodies. For the method for preparing an antibody, reference could be made to a conventional technical manual in the art (Howard, Gary C., and Matthew R. Kaser, eds. Making and using antibodies: a practical handbook. CRC press, 2013.).

In this example, the polypeptide used to prepare the antibody was specifically LDQPDPGAVAAAAIFRAILEVLQTQGA (SEQ ID NO: 7) linked to BSA (i.e., X was BSA), which was linked via an adapter Cys (C), referred to as LC28-BSA. The antibody was a monoclonal antibody. Of course, those skilled in the art should understand that this example is only used as an example, and the antibodies protected by the present disclosure are not limited to the antibodies shown in this example. Those skilled in the art can prepare antibodies for the aforementioned other polypeptides based on common sense and technical means.

In this example, the method for preparing an antibody based on LC28-BSA specifically included the following steps:

### (1) Animal immunization:

For the first immunization, LC28-BSA was emulsified with Freund's complete adjuvant and injected subcutaneously in the abdomen and footpad of Balb/C mice at multiple points, 50-100 µg per mouse, with a total dose of 0.2 mL/mouse. The second immunization was carried out 21 days later, and Freund's incomplete adjuvant was used for the second immunization, with a dose of 25-50 µg/0.2 mL/mouse. After the second immunization, immunization was carried out once every 2 weeks. After the fourth immunization, serum was collected for ELISA testing, and cell fusion could be performed when the titer reached 1:20,000.

Three days before fusion, 50 µg of the corresponding target recognition peptide (LC28) was injected intraperitoneally for sprint immunization.

### (2) Cell fusion:

Sacrifice immunized mice by cervical dislocation (IACUC-approved method), and the spleens were taken out, ground and crushed, and macromolecular impurities were removed using cell strainers. The splenocytes were then washed several times with RPMI-1640, and the splenocytes were counted. SP2/0 cells (mouse myeloma cells) that were in good condition in the logarithmic growth phase were added to spleen cells of immunized mice at a ratio of 1: 5-10, mixed thoroughly and centrifuged, and the supernatant was discarded. To the precipitated cells, 1 mL of 45% PEG solution was added, the centrifuge tube was gently rotated to allow cells to fully and evenly contact PEG, and allow to stand for 1 min; 1, 2, 3, 4, 5, and 10 mL of RPMI-1640 were added respectively every 2 min to terminate the PEG reaction, then allowed to stand for 10 min, centrifuged at 800 rpm for 5 min to collect the cell pellets. The cells were resuspended in HAT medium (Hypoxanthine, aminopterin, and thymidine medium) and inoculated into 96-well plates for continued culture. When the hybridoma cells grew to occupy about 1/10 of the well area, ELISA experiments were performed to screen positive hybridoma cells. After four times subcloning, a cell line that stably secreted monoclonal antibodies was obtained.

The cell line was acclimated with serum-free medium, the culture supernatant was collected, and the antibody in the supernatant was enriched and purified using protein A/G magnetic beads to obtain a monoclonal antibody (KA-001).

The prepared monoclonal antibody (KA-001) was sequenced, and its heavy chain amino acid sequence (Mouse IgG1 subtype) was as follows:

DVKIVESGGGLVKLGGSLKLSCAASGFTFSRNYMSWVRQTPEKRLELVAGINNN
GGSTYYPDTVKGRFTISRDNAKNTLYLQMSSLRSEDTAIYYCAKRGFITTVGAMDYW
GQGISVTVSSAKTTPPSVYPLAPGSAAQTNSMVTLGCLVKGYFPEPVTVTWNSGSLSSG
VHTFPAVLQSDLYTLSSSVTVPSSTWPSETVTCNVAHPASSTKVDKKIVPRDCGCKPCIC
TVPEVSSVFIFPPKPKDVLTITLTPKVTCVVVDISKDDPEVQFSWFVDDVEVHTAQTQPR
EEQFNSTFRSVSELPIMHQDWLNGKEFKCRVNSAAFPAPIEKTISKTKGRPKAPQVYTIP
PPKEQMAKDKVSLTCMITDFFPEDITVEWQWNGQPAENYKNTQPIMDTDGSYFVYSKL
NVQKSNWEAGNTFTCSVLHEGLHNHHTEKSLSHSPGK (SEQ ID NO: 19).

The bold and underlined parts were the three complementarity-determining regions (CDRs) in the heavy chain variable region of the antibody.

The nucleotide sequence corresponding to the heavy chain of KA-001:

5’-GATGTGAAGATAGTGGAAAGTGGTGGTGGATTGGTCAAACTTGGGGGGTCCCTG
AAACTCAGCTGCGCTGCCAGCGGCTTCACATTTAGTAGAAACTACATGTCCTGGGTG
AGGCAAACGCCAGAGAAGCGGCTGGAGCTTGTAGCTGGCATCAACAACAATGGAG
GCAGCACCTACTATCCTGACACAGTCAAAGGACGCTTCACCATATCCAGAGACAATG
CAAAGAATACCCTGTATCTGCAGATGTCATCTTTACGATCAGAAGATACTGCAATCTA
TTACTGTGCCAAGAGGGGTTTTATTACAACTGTGGGGGCCATGGACTACTGGGGCCA
GGGCATTTCTGTTACTGTGTCCTCCGCCAAGACCACACCACCTTCCGTCTATCCCTTG

GCACCAGGATCAGCCGCCCAAACTAACAGTATGGTGACACTGGGATGCCTGGTTAA
GGGCTATTTCCCTGAGCCAGTGACAGTGACTTGGAACAGTGGTAGCCTGAGTTCAG
GGGTCCACACTTTCCCCGCTGTCTTGCAGAGTGATCTGTATACCCTCAGCTCCTCAG
TGACCGTTCCTAGCAGTACGTGGCCTTCCGAGACTGTGACATGTAATGTAGCCCATC
CCGCTTCATCTACCAAGGTGGATAAGAAGATTGTTCCACGGGACTGCGGGTGTAAAC
CGTGTATTTGTACTGTACCCGAGGTGAGTTCCGTTTTCATTTTCCCTCCCAAGCCCAA
AGATGTTCTCACAATTACTCTGACACCAAAAGTTACATGCGTGGTGGTGGACATCAG
CAAGGACGATCCTGAGGTGCAGTTTTCCTGGTTTGTTGATGACGTCGAAGTCCACAC
TGCACAGACCCAGCCAAGGGAAGAACAGTTCAATAGTACATTCAGATCCGTGTCCG
AATTGCCGATTATGCATCAGGACTGGCTCAATGGCAAAGAATTTAAGTGTAGAGTGA
ACTCTGCCGCCTTTCCTGCTCCTATCGAAAAGACAATCAGCAAGACTAAGGGCAGA
CCTAAAGCCCCCCAAGTGTATACTATTCCACCCCCCAAAGAACAGATGGCCAAGGAC
AAAGTAAGTTTGACCTGTATGATCACTGACTTCTTCCCAGAAGATATCACTGTGGAAT
GGCAGTGGAACGGACAGCCTGCGGAGAACTATAAAAATACTCAGCCCATTATGGAC
ACAGACGGGTCTTACTTCGTCTACAGTAAACTGAATGTTCAGAAAAGTAATTGGGAG
GCCGGCAATACATTCACCTGTTCAGTGCTGCATGAGGGGCTTCACAACCACCACACT
GAGAAATCCCTTAGCCACAGCCCTGGCAAGTAA-3'(SEQ ID NO: 20).

The light chain amino acid sequence (Mouse Kappa subtype) of KA-001 was:

DIQMTQTTFSLSASLGDRVTISCRASQDISNYLNWYQQIPDGTVKLLIYYTSRLHS
GVPSRFSGSGSGTDYSLTISNLEQEDIATYFCQQVHTLPYTFGGGTKLEMKRADAAPTV
SIFPPSSEQLTSGGASVVCFLNNFYPKDINVKWKIDGSERQNGVLNSWTDQDSKDSTYS
MSSTLTLTKDEYERHNSYTCEATHKTSTSPIVKSFNRNEC(SEQ ID NO: 21).

The bold and underlined parts were the three complementarity-determining regions (CDRs) in the light chain variable region of the antibody.

The nucleotide sequence corresponding to the light chain of KA-001:

5’-GACATTCAGATGACCCAGACGACCTTCAGCCTCTCTGCATCCCTTGGAGACAGAG
TCACCATCAGCTGTCGGGCCAGCCAAGATATATCAAATTATCTGAACTGGTACCAGC
AGATCCCAGATGGCACTGTGAAGCTGCTTATCTACTACACATCACGCCTACATTCTGG
TGTTCCTTCCAGGTTTTCTGGGAGTGGCAGTGGGACAGACTATTCCCTCACCATTAG
CAACCTGGAGCAGGAAGATATTGCTACTTACTTCTGCCAGCAAGTGCACACACTGCC
CTATACTTTTGGAGGTGGCACCAAATTGGAGATGAAACGGGCCGATGCTGCTCCTAC
CGTCAGTATCTTCCCACCTTCAAGCGAACAGCTTACCAGTGGCGGGGCATCTGTGGT
GTGCTTCCTGAATAACTTTTACCCAAAGGATATAAACGTGAAGTGGAAGATCGACGG
CAGCGAGCGGCAAAATGGAGTGTTGAATAGTTGGACTGACCAGGACTCTAAGGACT
CCACCTACTCTATGTCATCTACACTTACACTGACAAAGGATGAGTACGAACGGCACA
ACTCCTATACATGCGAAGCTACCCATAAGACCAGTACCAGCCCCATTGTGAAGTCATT
CAATAGAAATGAGTGCTAA-3’(SEQ ID NO: 22).

### Example 2 Preparation and purification of antibodies based on genetic engineering

In the present disclosure, the acquisition of antibodies is not limited to a specific method. In this example, the method for preparing the KA-001 antibody based on genetic engineering method was exemplified.

The procedure was as follows:

### (1) Primer design:

In this example, the following primers were used for the preparation of KA-001 antibody by genetic engineering methods.

KA-001 Heavy Chain Primer Pair:
Upstream primer KA-001H-F: 5'-GGCCTCCGGACTCTAGAGGATCCGCCACCATGGAGACAGACACACTCCTGCT-3' (SEQ ID NO: 23);
Downstream primer KA-001H-R: 5'-CTCGAGCTAAGCTTCGAATTCTTACTTGCCAGGGCTGTG-3' (SEQ ID NO: 24).

KA-001 Light Chain Primer Pair:
Upstream primer KA-001L-F: 5'-CAGAATTCGAAGCTTAGCTCGAGAGCCACCATGGAGACAGACACACTCCTGCTAT GG-3' (SEQ ID NO: 25);
Downstream primer KA-001L-R: 5'-CAGAGGTTGATTGTCGAGATATCAAACTCATTACTAACCGGT-3' (SEQ ID NO: 26).

### (2) Acquisition of the target sequence:

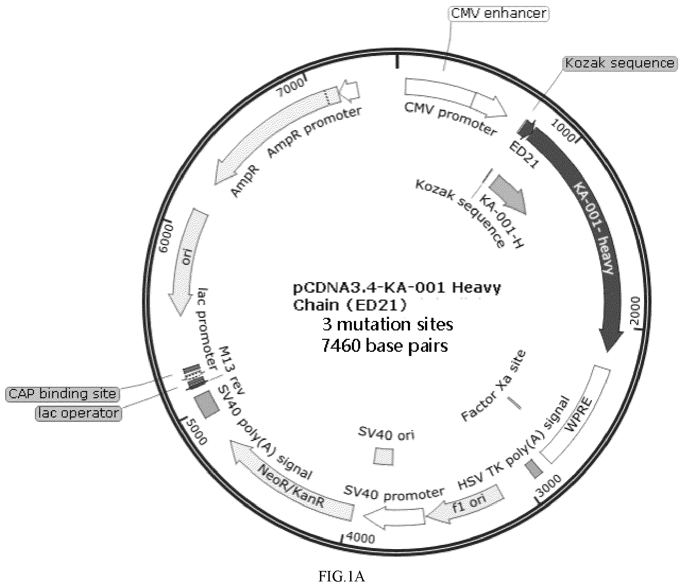

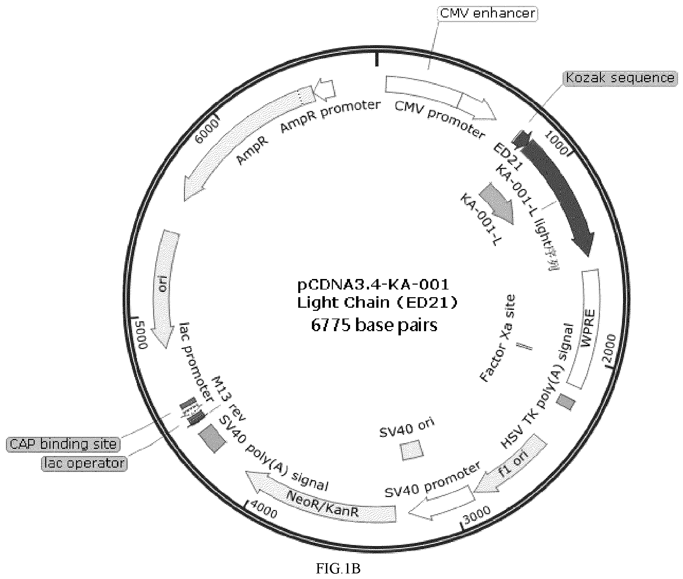

The plasmid containing the nucleic acid molecules encoding the sequences shown in SEQ ID NO: 20 and SEQ ID NO: 22 (the plasmid map was shown in FIG. 1, and the plasmid was named pcDNA3.4) was used as a template, and the target sequence was amplified based on the primers shown in SEQ ID NO: 23 to SEQ ID NO: 26 and PrimeSTAR high-fidelity enzyme. The amplification system and conditions could be adjusted according to the actual situation and the instructions for use of the relevant kit.

The amplified products were verified by 1% agarose gel electrophoresis. After confirmation, the target sequence was recovered.

### (3) Enzyme digestion and ligation of target sequence and vector plasmid:

The recovered target sequence and pcDNA3.4 vector were double-digested, and the restriction endonucleases NotI and XhoI were selected. A conventional enzyme digestion system in the art was used or restriction endonucleases were constructed with reference to the instructions. After double enzyme digestion, verification was performed using 1% agarose gel electrophoresis. After confirmation, the target sequence and linearized vector after restriction digestion were recovered.

The target sequence and the linearized vector were connected to obtain a recombinant vector.

(4) The recombinant vector obtained in (3) was used to transform DH5α competent cells, positive cells were screened (verified by PCR and sequencing), and plasmids were extracted.

(5) The plasmid extracted in (4) was transfected into expression vector cells (HEK-293) using KPM cell transfection buffer and TA-293 transfection reagent based on transient transfection technology. Cell protein expression enhancer (KE-293) and transient transfection nutrient additive (KT-Feed 50×) were added 24 hours after transfection to promote expression. The cell supernatant was collected 6 days after transfection, and the expression level of the target protein (KA-001) was determined by ELISA.

(6) The target protein in the cell supernatant was purified by liquid chromatography. The cell supernatant was centrifuged at 8000 rpm for 30 min and filtered through a 0.45 µm filter membrane. The filtrate was added to a 1-mL chromatography column at a flow rate of 1 mL/min. The column was washed with 10-15 column volumes of PBS to remove impurities and then eluted with a 0.1 M glycine solution at pH 3.0 to recover the target antibody based on the absorption peak. The recovered antibodies were neutralized with 1/10 volume of 1M Tris-HCl (pH 8.5). After ultrafiltration and concentration, the antibody concentration was determined by protein electrophoresis.

Meanwhile, the KA-004 antibody was prepared by the same genetic engineering method as described in the above example. The polypeptide corresponding to the KA-004 antibody was specifically EQPDPGAVAAAAILRAILEVLQS (SEQ ID NO: 12) linked to BSA (i.e., X was BSA), and BSA was linked to SEQ ID NO: 12 via an adapter Cys, referred to as EC24-BSA.

### Example 3 Specific binding effects of KA-001 and KA-004 on VLQS

The specific binding effect of KA-001 and KA-004 on VLQS was determined by ELISA method. The specific steps were as follows:
Peptide fragments KL19 (KEQPDPGAVAAAAILRAIL (SEQ ID NO: 27)), KL22 (KEQPDPGAVAAAAILRAILEVL (SEQ ID NO: 28)), and KS24 (KEQPDPGAVAAAAILRAILEVLQS (SEQ ID NO: 29)) were used, where KL19 did not contain VLQS, KL22 only contained VL, and KS24 contained VLQS. The three peptide fragments were used as coating antigens, and the binding of KA-001 and KA-004 to the three peptide fragments was detected by indirect ELISA.

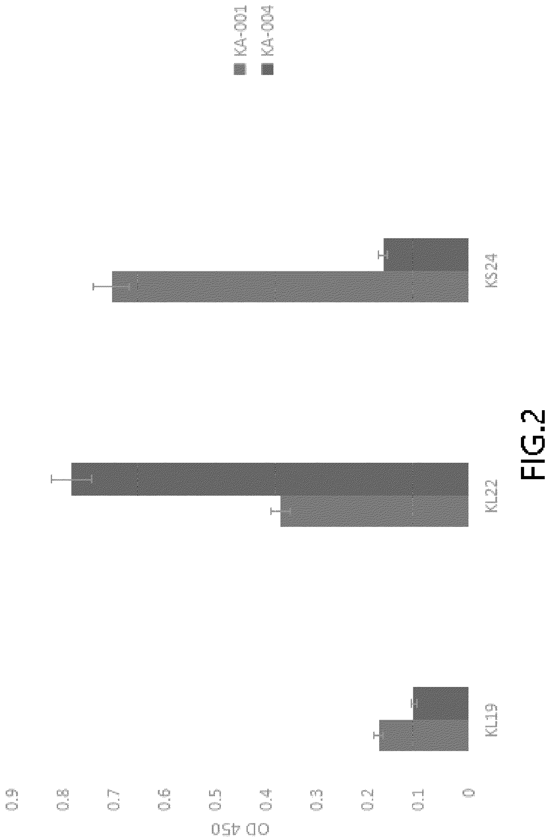

The results are shown in FIG. 2. It could be found that KA-001 and KA-004 could specifically bind to KS24 (the last two amino acids in VLQS) and its adjacent epitope KL22 (the first two amino acids in VLQS), respectively, but neither bound to KL19, indicating that the recognition epitope of the antibody in the above example was an amino acid sequence with more than 50% identity in VLQ. The inventors found that after the antibody recognizing the epitope formed a self-assembled probe with the targeting polypeptide, it has a more sensitive recognition ability for immune cells.

### Example 4 Self-assembly of antibodies and target recognition polypeptides could enhance the specific recognition ability of antibodies to immune cells and the fluorescent staining intensity

In this example of the present disclosure, the inventors used fluorescent group-modified KA-001 as an example to verify that the self-assembled composition of the antibody targeting the polypeptide and the polypeptide could be used to target, identify or label immune cells.

Of course, those skilled in the art should understand that this embodiment is only used as an example, and the effect is not limited to KA-001 monoclonal antibody.

The specific experimental steps were:
Alexa Fluor 647 was used to modify KA-001. The specific modification method was as follows: 1 mL of 2.54 mg/mL KA-001 antibody was taken and dialyzed with 0.1 M sodium bicarbonate for 4 h, and then 165 µL of Alexa Fluor 647-DMSO solution (a mixture containing Alexa Fluor 647 with a final concentration of 10 mg/mL prepared using DMSO as a solvent) was added, reacted for 1 h, and dialyzed with PBS for 48 h to obtain Alexa Fluor 647-modified KA-001.

Two groups of healthy Balb/c mice were selected and their peripheral blood was collected. 50 µL of mouse peripheral blood was collected in EP tubes, and ES23 with a final concentration of 10 µM (specific sequence: EQPDPGAVAAAAILRAILEVLQS (SEQ ID NO: 30))/SS28 (specific sequence: SSARLEQPDPGAVAAAAILRAILEVLQS (SEQ ID NO: 31)), Alexa Fluor 647-modified KA-001 at a final concentration of 0.01 mg/mL were added. After thorough mixing, the mixture was incubated at 4 °C for 30 min. The peripheral whole blood to which antibody-specific recognition polypeptides ES23/SS28 and Alexa Fluor 647-modified KA-001 were added was lysed with 0.4% (m/m) ammonium chloride (prepared with pure water and filtered through 0.1 µm before use) at a volume ratio of 1:25 (ammonium chloride: peripheral whole blood). After lysis at room temperature for 10 min, the blood was centrifuged at 400 g for 5 min, and the precipitated cells were collected, resuspended in PBS, centrifuged at 400 g for 5 min, and washed twice. Finally, the cells were resuspended in 100 µL of PBS and analyzed by flow cytometry.

The group without the addition of antibody-specific recognition polypeptide was used as a control. The experiment was repeated twice.

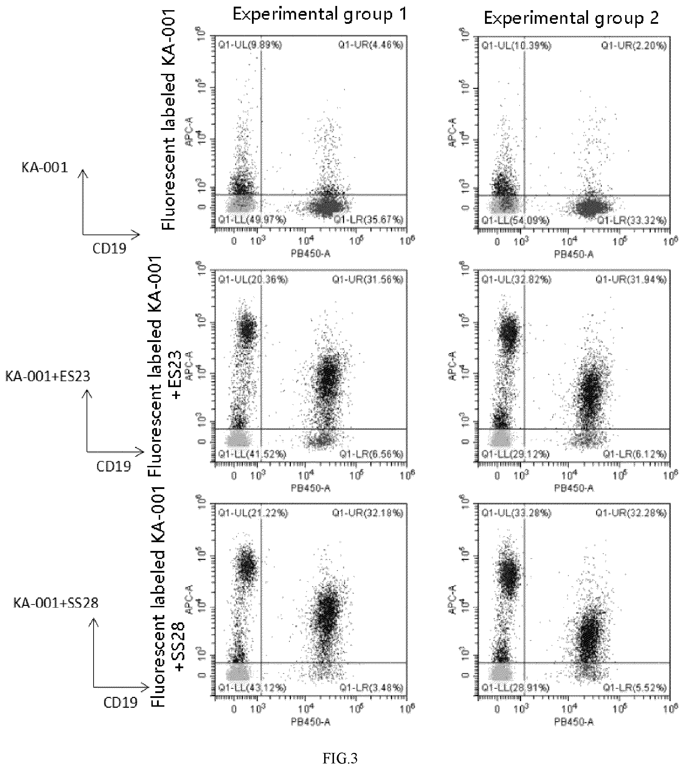

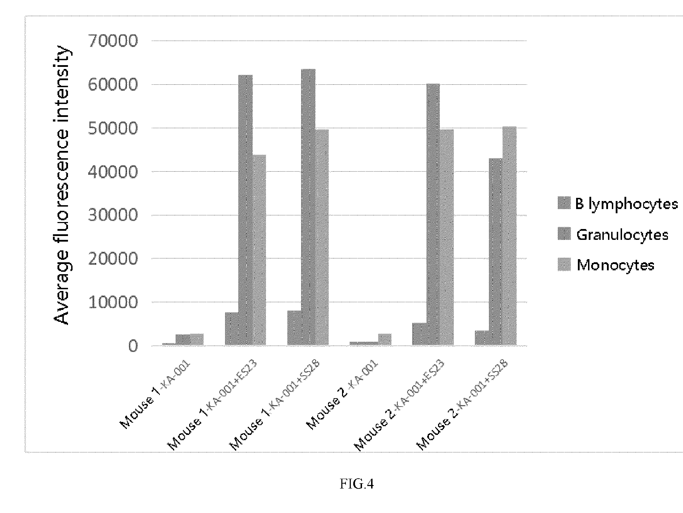

The results are shown in FIG. 3 and FIG. 4.

It could be found that, after two repeated experiments, KA-001 modified with fluorescent groups alone had no obvious binding effect on peripheral blood cells of healthy mice, and its positive percentage in identifying monocytes, neutrophils, and B lymphocytes was low. After further adding antibody-specific recognition polypeptides, the self-assembled composition significantly enhanced the recognition of monocytes, neutrophils, and B lymphocytes, and the positive percentage increased, indicating that its binding effect had been significantly improved compared to KA-001 modified with fluorescent groups alone.

To further confirm the above conclusion, the inventors repeated the above experiment using KA-004 instead of KA-001.

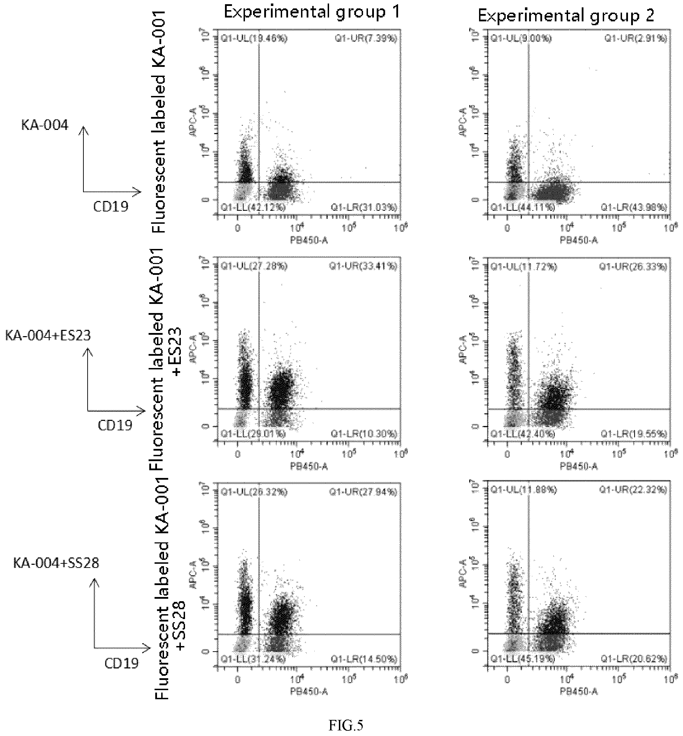

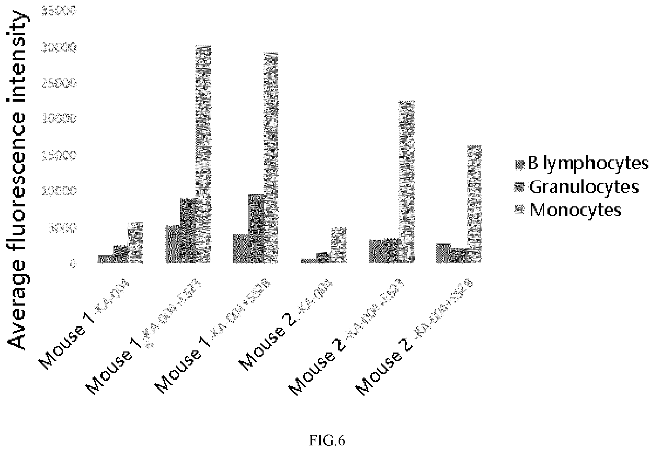

The results are shown in FIG. 5 and FIG. 6.

Results demonstrated that when KA-004 replaced KA-001, the self-assembled composition formed by adding antibody-specific recognition polypeptides exhibited significantly higher recognition efficiency for neutrophils and B lymphocytes than KA-004 modified with fluorescent groups alone. This confirmed that the antibody and its specific recognition polypeptide disclosed herein could significantly improve the antibody's targeting specificity toward immune cells and effectively enhance visual discrimination (based on fluorescence intensity).

The foregoing examples are preferred embodiments of the present disclosure, but the embodiments of the present disclosure are not limited to the above examples. Any other changes, modifications, substitutions, combinations, and simplifications made without departing from the spirit and principles of the present disclosure shall be equivalent replacement methods and shall be included in the protection scope of the present disclosure.

## Claims

1. An antibody or a functional fragment thereof, wherein the antibody or the functional fragment thereof targets and recognizes a C-terminal fragment sequence of Triokinase/FMN cyclase; and
wherein the C-terminal fragment sequence of Triokinase/FMN cyclase comprises:
(a) an amino acid sequence containing VLQ motif;
(b) an amino acid sequence with at least 80% sequence identity to the amino acid sequence in (a), wherein the amino acid sequence is of non-human animal origin; and
(c) a variant of the amino acid sequence of (a) or (b).

2. The antibody or the functional fragment thereof according to claim 1, which targets and recognizes the C-terminal fragment sequence of Triokinase/FMN cyclase, wherein the amino acid sequences of (a), (b) and (c) comprise 45 or less amino acid residues, preferably comprise 41 or less amino acid residues, and more preferably comprise 9 to 41 amino acid residues.

3. The antibody or the functional fragment thereof according to claim 1, wherein the C-terminal fragment sequence of Triokinase/FMN cyclase described in (a) comprises an amino acid sequence with at least 80% sequence identity to any one of SEQ ID NOs: 2-18 or 29.

4. The antibody or the functional fragment thereof according to claim 1, wherein the variant comprises a composite polypeptide obtained by ligating the C-terminal fragment sequence of Triokinase/FMN cyclase with an adapter, a marker, a tag, an amino acid sequence or a carrier, and the variant is immunogenic.

5. The antibody or the functional fragment thereof according to claim 4, wherein the carrier comprises a protein carrier, and the protein carrier comprises hemocyanin, serum albumin, and ovalbumin.

6. The antibody or the functional fragment thereof according to any one of claims 1 to 4, wherein amino acid sequences of complementarity-determining regions (CDRs) of a heavy chain variable region of the antibody are shown in the amino acid sequence at positions 26 to 35, 50 to 66 and 99 to 110 of SEQ ID NO: 19, respectively; amino acid sequences of a complementarity-determining regions (CDRs) of a light chain variable region is shown in the amino acid sequence at positions 24 to 34, 50 to 56 and 89 to 97 of SEQ ID NO: 21, respectively.

7. The antibody or the functional fragment thereof according to claim 6, wherein the heavy chain variable region of the antibody is shown in SEQ ID NO: 19, and the light chain variable region is shown in SEQ ID NO: 21.

8. The antibody or the functional fragment thereof according to any one of claims 6 to 7, wherein the antibody further comprises a constant region, and the constant region is preferably a human constant region.

9. The antibody or the functional fragment thereof according to claim 1, wherein the antibody is modified or linked with a small-molecule active substance.

10. The antibody or the functional fragment thereof according to claim 9, wherein the small-molecule active substance comprises a polypeptide adapter, a biotin-(strept)avidin system, a chromogenic enzyme, a fluorescent labeling group, a chemiluminescent labeling group, an isotope or a magnetic functional group;
the adapter comprises lysine (K) and cysteine (C);
the chromogenic enzyme comprises peroxidase and alkaline phosphatase;
the fluorescent labeling group comprises fluorescent proteins, rhodamines, fluoresceins, indocyanines, cyanine dyes, Alexa Fluor dyes and/or quantum dot fluorescent groups;
the magnetic functional group comprises a group capable of magnetic resonance imaging and changing relaxation efficiency; and
the isotope comprises a radionuclide.

11. The antibody or the functional fragment thereof according to claim 10, wherein the antibody targets and recognizes immune cells; preferably, the immune cells comprise but is not limited to lymphocytes, dendritic cells, monocyte precursors, monocytes/macrophages, neutrophils, basophils, eosinophils and mast cells.

12. A nucleic acid molecule encoding the antibody or the functional fragment thereof according to any one of claims 1 to 11.

13. An expression vector or transformant containing the nucleic acid molecule of claim 12.

14. A probe targeting immune cells, comprising the antibody or the functional fragment thereof and an antigen polypeptide thereof according to any one of claims 1 to 11.

15. The probe according to claim 14, wherein the recognition ability and sensitivity of the antibody to immune cells are enhanced after the antibody is self-assembled with the antigen polypeptide thereof.

16. Use of the antibody or the functional fragment thereof according to any one of claims 1 to 11, the nucleic acid molecule of claim 12, or the expression vector or transformant of claim 13 in preparation of a product for targeting immune cells.

17. The use according to claim 16, wherein the product comprises a probe, a detection reagent, a detection kit, and a detection chip.

18. The use according to claim 16, wherein the immune cells comprise but not limited to lymphocytes, dendritic cells, monocyte precursors, monocytes/macrophages, neutrophils, basophils, eosinophils, and mast cells.
